# EUROPEAN PATENT APPLICATION

(11) **EP 2 514 366 A1**
(43) Date of publication of application: **24.10.2012**
(21) Application number: 12156359.7
(22) Date of filing: 21.02.2012
(51) Int. Cl.: A61B 8/00

(54) **Automatic ultrasonic scanning system and scanning method thereof**

(30) Priority: 22.04.2011 TW 100114164
(71) Applicant: Li, Pai-Chi, Taipei 10617 (TW)
(72) Inventor: Li, Pai-Chi, Taipei 10617 (TW)
(74) Representative: Reichert, Sabine

(57) **Abstract**

An exemplary automatic ultrasonic scanning system and a scanning method of the same are disclosed. The automatic ultrasonic scanning system includes a multi-axis robot arm (104), an ultrasonic scan head (108) disposed on the multi-axis robot arm, a control circuit (102) for controlling the multi-axis robot arm, a three-dimensional image capturing apparatus (110) and a computer (106). The computer senses a tested object through the three-dimensional image capturing apparatus, creates a three-dimensional shape of the tested object, and plans a three-dimensional scanning path according to the three-dimensional shape. According to the three-dimensional scanning path the computer further controls the multi-axis robot arm to perform a multi-axis motion through the control circuit, so as performs a three-dimensional scan on the tested object through the ultrasonic scan head, and constructs an ultrasonic image according to a reflected ultrasonic signal received by the ultrasonic scan head consequently.

## Description

### FIELD OF THE INVENTION

The present invention generally relates to a medical equipment, and more particularly to an automatic ultrasonic scanning system and a scanning method thereof.

### BACKGROUND OF THE INVENTION

Ultrasonic scan is a very important tool for the medical inspection. The existing ultrasonic inspection items primarily include the general ultrasonic inspection, the obstetric ultrasonic inspection, the heart ultrasonic inspection, the vascular ultrasonic inspection, and so on. Today, the ultrasonic scan applications are not only applied to the diagnosis field but also applied to the screening field.

However, the conventional ultrasonic inspection is necessary to be manually performed by doctors or technical persons, and the manual inspection manner is relatively inefficient for the disease screening. Besides, the manual inspection manner much relies on the operator's education, scanning technique and experience. Therefore, once any mistakes are happened in the scanning process, the improper ultrasonic scan may lead to a creation of an unclear scanning image or even lead a doctor to make a wrong inspection.

### SUMMARY OF THE INVENTION

Therefore, an objective of the present invention is to provide an automatic ultrasonic scanning system capable of automatically performing an ultrasonic scan, thereby the risk of resulting error factors in a human operation is reduced.

Another objective of the present invention is to provide a scanning method applied to the aforementioned automatic ultrasonic scanning system.

The present invention discloses an automatic ultrasonic scanning system comprising a control circuit, a multi-axis robot arm, a computer, an ultrasonic scan head and a three-dimensional image capturing apparatus. The ultrasonic scan head is disposed on the multi-axis robot arm. The control circuit is configured to control the multi-axis robot arm. The computer is electrically coupled to the three-dimensional image capturing apparatus, the control circuit and the ultrasonic scan head. The computer is configured to sense a tested object through the three-dimensional image capturing apparatus, create a three-dimensional shape of the tested object based on the sensed result, and plan a three-dimensional scanning path according to the three-dimensional shape. According to the three-dimensional scanning path the computer further controls the multi-axis robot arm to perform a multi-axis motion through the control circuit, so as a three-dimensional scan is performed on the tested object by the ultrasonic scan head, thereby an ultrasonic image is constructed according to a reflected ultrasonic signal received by the ultrasonic scan head consequently.

In one embodiment, the above mentioned three-dimensional image capturing apparatus comprises a depth camera. The depth camera is used for creating a depth map of the tested object; thereby the computer can create the three-dimensional shape according to a position and a corresponding depth of each of a plurality of points on the depth map.

In one embodiment, the above mentioned computer computes a normal vector of a plan which is formed by any three of the points according to the position and the corresponding depth of each of the points on the depth map, thereby the computer can further plan the three-dimensional scanning path and a rotating angle of the ultrasonic scan head according to the normal vector of each of the planes and the corresponding depth of each of the points on the depth map.

In one embodiment, the above mentioned computer further refers the point on the depth map having a maximum depth value to a motion reference point of the multi-axis robot arm.

In one embodiment, the above mentioned ultrasonic scan head is driven by the computer.

In one embodiment, the above mentioned multi-axis robot arm is a six-axis robot arm.

In one embodiment, the above mentioned computer further performs an assisting diagnostic operation on the ultrasonic image.

In one embodiment, the above mentioned three-dimensional image capturing apparatus comprises at least one camera for creating a plurality of two-dimensional images of the tested object, thereby the computer can further create the three-dimensional shape through performing a three-dimensional space constructive operation on these two-dimensional images.

The present invention also discloses a scanning method of an automatic ultrasonic scanning system implemented by a multi-axis robot arm, a three-dimensional image capturing apparatus and an ultrasonic scan head disposed on the multi-axis robot arm. The scanning method comprises steps of: creating a three-dimensional shape of a tested object through the three-dimensional image capturing apparatus sensing the tested object; planning a three-dimensional scanning path according to the three-dimensional shape; controlling the multi-axis robot arm to perform a multi-axis motion according to the three-dimensional scanning path and performing a three-dimensional scan on the tested object through the ultrasonic scan head; and constructing an ultrasonic image according to a reflected ultrasonic signal received by the ultrasonic scan head.

In one embodiment, the above mentioned three-dimensional image capturing apparatus comprises a depth camera for creating a depth map of the tested object. The scanning method further comprises a step of: creating the three-dimensional shape according to a position and a corresponding depth of each of a plurality of points on the depth map.

In one embodiment, the above mentioned scanning method further comprises a step of: computing a normal vector of a plan which is formed by any three of the points according to the position and the corresponding depth of each of the points on the depth map, and further planning the three-dimensional scanning path and a rotating angle of the ultrasonic scan head according to the normal vector of each of the planes and the corresponding depth of each of the points.

In one embodiment, the above mentioned scanning method further comprises a step of: referring the point on the depth map having a maximum depth value to a motion reference point of the multi-axis robot arm.

In one embodiment, the above mentioned scanning method further comprises a step of: performing an assisting diagnostic operation on the ultrasonic image.

In one embodiment, the above mentioned three-dimensional image capturing apparatus comprises at least one camera for creating a plurality of two-dimensional images of the tested object. The scanning method further comprises a step of: creating the three-dimensional shape through performing a three-dimensional space constructive operation on the two-dimensional images.

In summary, the present invention discloses an automatic ultrasonic scanning system which is implemented by a control circuit, a multi-axis robot arm, a computer, an ultrasonic scan head and a three-dimensional image capturing apparatus. The ultrasonic scan head is disposed on the multi-axis robot arm. The control circuit is configured to control the multi-axis robot arm. The computer is electrically coupled to the three-dimensional image capturing apparatus, the control circuit and the ultrasonic scan head. In the present invention, the computer is configured to sense a tested object through the three-dimensional image capturing apparatus, create a three-dimensional shape of the tested object based on the sensed result, and plan a three-dimensional scanning path according to the three-dimensional shape. According to the three-dimensional scanning path the computer further controls the multi-axis robot arm to perform a multi-axis motion through the control circuit, so as a three-dimensional scan is performed on the tested object by the ultrasonic scan head, thereby an ultrasonic image is constructed according to a reflected ultrasonic signal received by the ultrasonic scan head consequently. Because the automatic ultrasonic scanning system can automatically perform the ultrasonic scan based on a three-dimensional scanning path, the risk of resulting error factors in the humane operation is reduced consequently.

### BRIEF DESCRIPTION OF THE DRAWINGS

The above objects and advantages of the present invention will become more readily apparent to those ordinarily skilled in the art after reviewing the following detailed description and accompanying drawing, in which:

FIG. 1 is a schematic block diagram of an automatic ultrasonic scanning system in accordance with a preferred embodiment of the present invention; and

FIG. 2 is a flow chart of a scanning method of the automatic ultrasonic scanning system in accordance with a preferred embodiment of the present invention.

### DETAILED DESCRIPTION OF PREFERRED EMBODIMENTS

The present invention will now be described more specifically with reference to the following embodiments. It is to be noted that the following descriptions of preferred embodiments of this invention are presented herein for purpose of illustration and description only. It is not intended to be exhaustive or to be limited to the precise form disclosed.

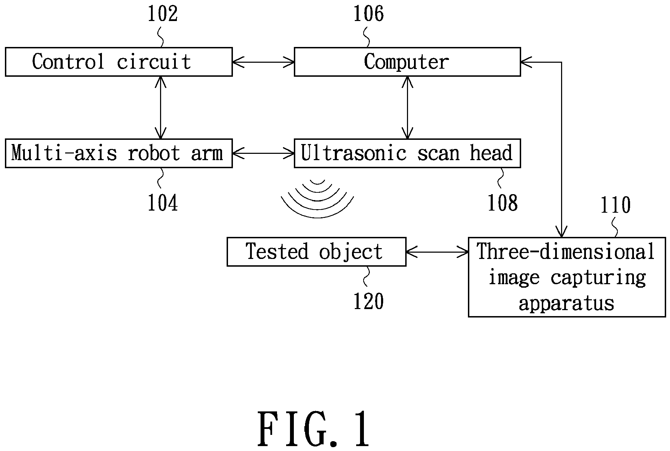

**FIG. 1** is a schematic block diagram of an automatic ultrasonic scanning system in accordance with a preferred embodiment of the present invention. As depicted in **FIG. 1****,** the automatic ultrasonic scanning system comprises a control circuit 102, a multi-axis robot arm 104 (e.g., a six-axis robot arm), a computer 106, an ultrasonic scan head 108 and a three-dimensional image capturing apparatus 110. The ultrasonic scan head 108 is disposed on the multi-axis robot arm 104; thereby the ultrasonic scan head 108 can perform an ultrasonic scan on a tested object 120 along with a movement of the multi-axis robot arm 104. The control circuit 102 is configured to control the multi-axis robot arm 104 to perform a specific movement, such as a moving, a rotating or even a telescopic movement. The computer 106 is electrically coupled to and configured to control the three-dimensional image capturing apparatus 110, the control circuit 102 and the ultrasonic scan head 108.

The computer 106 is configured to sense the tested object 120 (such as a woman's breast) through the three-dimensional image capturing apparatus 110, so as a three-dimensional shape of the tested object 120 is created based on the sensed result, thereby the computer 106 can plan a three-dimensional scanning path of the tested object 120 according to the three-dimensional shape. Moreover, through the control circuit 102 the computer 106 further controls the multi-axis robot arm 104 to perform a multi-axis motion according to the three-dimensional scanning path, so as the ultrasonic scan head 108 simultaneously performs a three-dimensional scan on the tested object 120 along with the multi-axis motion of the multi-axis robot arm 104, and an ultrasonic image is constructed according to a reflected ultrasonic signal received by the ultrasonic scan head 108 consequently.

The three-dimensional image capturing apparatus 110 can be implemented by a depth camera for creating a depth map of the tested object 120. Accordingly, the computer 106 firstly creates the aforementioned three-dimensional shape of the tested object 120 according to a position and a corresponding depth of each of the points on the depth map; consequently, based on the position and the corresponding depth of each of the points on the depth map the computer 106 computes a normal vector of a plan which is formed by any three of the points on the depth map; thereby the computer 106 plans the aforementioned three-dimensional scanning path and a rotating angle of the ultrasonic scan head 108 according to the normal vector of each of the planes and the corresponding depth of each of the points. It is indicated that the rotating angle of the ultrasonic scan head 108 is controlled by the rotating angle of the multi-axis robot arm 104. Besides, the computer 106 may further refer the point on the depth map having a maximum depth value to a motion reference point for a use of positioning for the multi-axis robot arm 104.

Preferably, the computer 106 may further be configured to perform an assisting diagnostic operation on the obtained ultrasonic image, thereby the disease screening efficiency is enhanced and the risk of resulting error factors in the humane operation is reduced consequently. Besides, the ultrasonic scan head 108 is driven by the computer 106 in the exemplary block diagram of the automatic ultrasonic scanning system as depicted in **FIG. 1****,** but it is not intended to limit the present invention.

Instead of the depth camera, it is indicated that the three-dimensional image capturing apparatus 110 can be implemented by at least one camera for creating at least two two-dimensional images of the tested object 120. Accordingly, the aforementioned three-dimensional image is created through configuring the computer 106 to perform a three-dimensional space reconstruction operation on these two two-dimensional images.

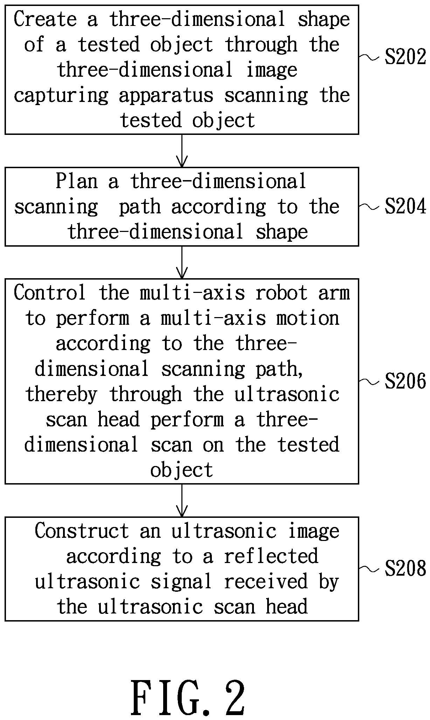

According to the aforementioned description related to the automatic ultrasonic scanning system of the present invention, the operation of the aforementioned automatic ultrasonic scanning system can be summarized to several basic operating steps as depicted in **FIG. 2. FIG. 2** is flow chart of a scanning method of the automatic ultrasonic scanning system in accordance with a preferred embodiment of the present invention. Based on the aforementioned description related to the automatic ultrasonic scanning system, the automatic ultrasonic scanning system comprises a multi-axis robot arm, a three-dimensional image capturing apparatus and an ultrasonic scan head which is disposed on the three-dimensional image capturing apparatus. As depicted in **FIG. 2****,** the scanning method primarily comprises steps of: create a three-dimensional shape of a tested object through controlling the three-dimensional image capturing apparatus to sense the tested object (step S202); plan a three-dimensional scanning path according to the aforementioned three-dimensional shape (step S204); control the multi-axis robot arm to perform a multi-axis motion according to the aforementioned three-dimensional scanning path so as the ultrasonic scan head performs a three-dimensional scan on the tested object (step S206); and construct an ultrasonic image according to a reflected ultrasonic signal received by the ultrasonic scan head (step S208).

Specifically, if the three-dimensional image capturing apparatus is implemented by a depth camera and a depth map of the tested object is created by the depth camera, the scanning method may further comprise steps of: create the aforementioned three-dimensional shape according to a position and a corresponding depth of each of a plurality of points on the depth map; and compute a normal vector of a plan which is formed by any three of the points according to the position and the corresponding depth of each of the points on the depth map, and further plan the aforementioned three-dimensional scanning path and a rotating angle of the ultrasonic scan head according to the normal vector of each of the planes and the corresponding depth of each of the points. In addition, the scanning method may further comprise a step of: refer the point on the depth map having a maximum depth value to a motion reference point of the multi-axis robot arm.

Specifically, if the three-dimensional image capturing apparatus is implemented by at least one camera, the scanning method may further comprise a step of: create the aforementioned three-dimensional shape through performing a three-dimensional space constructive operation on the two-dimensional images which are created by the at least one camera.

Besides, the scanning method may further comprise a step of: perform an assisting diagnostic operation on the ultrasonic image.

To sum up, the present invention discloses an automatic ultrasonic scanning system which is implemented by a control circuit, a multi-axis robot arm, a computer, an ultrasonic scan head and a three-dimensional image capturing apparatus. The ultrasonic scan head is disposed on the multi-axis robot arm. The control circuit is configured to control the multi-axis robot arm. The computer is electrically coupled to the three-dimensional image capturing apparatus, the control circuit and the ultrasonic scan head. In the present invention, the computer is configured to sense a tested object through the three-dimensional image capturing apparatus, create a three-dimensional shape of the tested object based on the sensed result, and plan a three-dimensional scanning path according to the three-dimensional shape. According to the three-dimensional scanning path the computer further controls the multi-axis robot arm to perform a multi-axis motion through the control circuit, so as a three-dimensional scan is performed on the tested object by the ultrasonic scan head, thereby an ultrasonic image is constructed according to a reflected ultrasonic signal received by the ultrasonic scan head consequently. Because the automatic ultrasonic scanning system can automatically perform the ultrasonic scan based on a three-dimensional scanning path, the risk of resulting error factors in the humane operation is reduced consequently.

While the invention has been described in terms of what is presently considered to be the most practical and preferred embodiments, it is to be understood that the invention needs not be limited to the disclosed embodiment. On the contrary, it is intended to cover various modifications and similar arrangements included within the scope of the appended claims which are to be accorded with the broadest interpretation so as to encompass all such modifications and similar structures.

## Claims

1. An automatic ultrasonic scanning system, **characterized in that** the automatic ultrasonic scanning system comprises:
a multi-axis robot arm (104);
an ultrasonic scan head (108), disposed on the multi-axis robot arm (104);
a control circuit (102), for controlling the multi-axis robot arm (104);
a three-dimensional image capturing apparatus (110); and
a computer (106), electrically coupled to the three-dimensional image capturing apparatus (110), the control circuit (102) and the ultrasonic scan head (108), wherein the computer (106) senses a tested object (120) through the three-dimensional image capturing apparatus (110), creates a three-dimensional shape of the tested object (120), and plans a three-dimensional scanning path according to the three-dimensional shape, according to the three-dimensional scanning path the computer (106) further controls the multi-axis robot arm (104) to perform a multi-axis motion through the control circuit (102), thereby performing a three-dimensional scan on the tested object (120) through the ultrasonic scan head (108), and constructs an ultrasonic image according to a reflected ultrasonic signal received by the ultrasonic scan head (108) consequently.

2. The automatic ultrasonic scanning system according to claim 1, **characterized in that** the three-dimensional image capturing apparatus (110) comprises a depth camera for creating a depth map of the tested object (120), and the computer (106) can create the three-dimensional shape according to a position and a corresponding depth of each of a plurality of points on the depth map.

3. The automatic ultrasonic scanning system according to claim 2, **characterized in that** the computer (106) computes a normal vector of a plan which is formed by any three of the points according to the position and the corresponding depth of each of the points on the depth map, thereby the computer (106) can further plan the three-dimensional scanning path and a rotating angle of the ultrasonic scan head (108) according to the normal vector of each of the planes and the corresponding depth of each of the points on the depth map.

4. The automatic ultrasonic scanning system according to claim 2, **characterized in that** the computer (106) further refers the point on the depth map having a maximum depth value to a motion reference point of the multi-axis robot arm (104).

5. The automatic ultrasonic scanning system according to claim 1, **characterized in that** the ultrasonic scan head (108) is driven by the computer (106).

6. The automatic ultrasonic scanning system according to claim 1, **characterized in that** the multi-axis robot arm (104) is a six-axis robot arm.

7. The automatic ultrasonic scanning system according to claim 1, **characterized in that** the computer (106) further performs an assisting diagnostic operation on the ultrasonic image.

8. The automatic ultrasonic scanning system according to claim 1, **characterized in that** the three-dimensional image capturing apparatus (110) comprises at least one camera for creating a plurality of two-dimensional images of the tested object (120), thereby the computer (106) can further create the three-dimensional shape through performing a three-dimensional space constructive operation on the two-dimensional images.

9. A scanning method of an automatic ultrasonic scanning system, the automatic ultrasonic scanning system including a multi-axis robot arm (104), a three-dimensional image capturing apparatus (110) and an ultrasonic scan head (108) disposed on the multi-axis robot arm (104), **characterized in that** the scanning method comprises steps of:
creating a three-dimensional shape of a tested object (120) through the three-dimensional image capturing apparatus (110) sensing the tested object (120);
planning a three-dimensional scanning path according to the three-dimensional shape;
controlling the multi-axis robot arm (104) to perform a multi-axis motion according to the three-dimensional scanning path and thereby performing a three-dimensional scan on the tested object (120) through the ultrasonic scan head (108); and
constructing an ultrasonic image according to a reflected ultrasonic signal received by the ultrasonic scan head (108).

10. The scanning method according to claim 9, **characterized in that** the three-dimensional image capturing apparatus (110) comprises a depth camera for creating a depth map of the tested object (120), the scanning method further comprises a step of:
creating the three-dimensional shape according to a position and a corresponding depth of each of a plurality of points on the depth map.

11. The scanning method according to claim 10 further comprising a step of:
computing a normal vector of a plan which is formed by any three of the points according to the position and the corresponding depth of each of the points on the depth map, and further planning the three-dimensional scanning path and a rotating angle of the ultrasonic scan head (108) according to the normal vector of each of the planes and the corresponding depth of each of the points.

12. The scanning method according to claim 10 further comprising a step of:
referring the point on the depth map having a maximum depth value to a motion reference point of the multi-axis robot arm (104).

13. The scanning method according to claim 10 further comprising a step of:
performing an assisting diagnostic operation on the ultrasonic image.

14. The scanning method according to claim 9, **characterized in that** the three-dimensional image capturing apparatus (110) comprises at least one camera for creating a plurality of two-dimensional images of the tested object (120), the scanning method further comprises a step of:
creating the three-dimensional shape through performing a three-dimensional space constructive operation on the two-dimensional images.
